# EUROPEAN PATENT SPECIFICATION

(11) **EP 2 090 224 B1**
(45) Date of publication and mention of the grant of the patent: **20.08.2014**
(21) Application number: 06833216.2
(22) Date of filing: 24.11.2006
(51) Int. Cl.: A61B 3/14, A61B 3/15

(54) **FUNDUS PHOTOGRAPHING DEVICE**
FUNDUS-FOTOGERÄT
DISPOSITIF DE PHOTOGRAPHIE DE FOND D'OEIL

(43) Date of publication of application: 19.08.2009
(73) Proprietor: Nidek Co., Ltd., Gamagori-shi Aichi 443-0038 (JP)
(72) Inventor: LIA, Cesario, Gamagori-shi Aichi 443-0038 (JP); VIOLA, Marco, Gamagori-shi Aichi 443-0038 (JP); UENO, Tokio, Gamagori-shi Aichi 443-0038 (JP)
(74) Representative: Prüfer & Partner GbR European Patent Attorneys
(86) International application number: PCT/JP2006/323412
(87) International publication number: WO 2008/062527

(56) References cited:
- JP-A- 11 146 864
- JP-A- 2002 291 701
- JP-A- 2004 290 535
- JP-A- 2004 290 535
- JP-A- 2005 312 751
- JP-A- 2005 312 751
- US-A1- 2002 159 028
- US-A1- 2005 275 804

## Description

### Technical Field

The present invention relates to a fundus photographing device for photographing a fundus of an examinee's eye.

### Background Art

A fundus camera for photographing an eye fundus has heretofore been known, which is moved by an operator to make alignment and focusing (focus) with respect to an examinee's eye (see Patent Literature 1, for example). Such fundus camera projects an alignment index and a focus index on the examinee's eye and the operator adjusts a position of the camera while observing the indexes and then photographs the fundus.

US 6733129 B2 describes an ophthalmologic apparatus and an aligning method for this ophthalmologic apparatus including a measurement optical system for measuring the eyes of an object to be examined and an illumination system for illuminating the eyes with extrinsic eye light. This apparatus allows alignment of a measurement optical system with respect to a patient's eye to be examined, and provides an auto-alignment method for the apparatus. Thus, a method and an ophthalmologic apparatus is suggested, which allow high precision auto-alignment in a wide range.

### Citation List

### Patent Literature

Patent Literature 1: JP11(1999)-4808A

### Summary of Invention

### Technical Problem

However, in the case where the aforementioned fundus camera is used to obtain a good fundus image, the operator needs to have some experience in position adjustment of the fundus camera. This is not easy.

The present invention has been made to solve the above problems and has a purpose to provide a fundus photographing device capable of making easy position adjustment of the device with respect to an examinee's eye and appropriately photographing an eye fundus.

### Solution to Problem

To achieve the above purpose, the invention is claimed in claim 1. The following configurations are disclosed.
(1) A fundus photographing device having a fundus photographing optical system for photographing a fundus of an examinee's eye, comprises: an alignment index projecting means that includes light sources for alignment placed symmetrically with respect to a photographing optical axis and is arranged to project a pair of alignment indexes onto the examinee's eye; an anterior segment photographing means that is arranged to photograph an anterior segment of the examinee's eye on which the alignment indexes are projected by the alignment index projecting means; a pupil center detecting means that is arranged to determine a center of a pupil of the examinee's eye by image processing of an image of the anterior segment photographed by the anterior segment photographing means; an alignment index detecting means that is arranged to determine a middle point of the pair of alignment indexes photographed by the anterior segment photographing means and an interval between the alignment indexes; an alignment information obtaining means that is arranged to obtain alignment information of the device in up and down, and left and right directions relative to the examinee's eye from the detected pupil center and the detected middle point of the pair of alignment indexes and obtain alignment information of the device in back and forth directions relative to the examinee's eye from the detected interval of the alignment indexes; and alignment means that is arranged to make alignment of the device based on the alignment information obtained by the alignment information obtaining means.
(2) In the fundus photographing device (1), the alignment index is a rectangular index extending in a vertical direction with respect to the examinee's eye.
(3) The fundus photographing device (1), further comprises: an illumination light irradiating optical system for illuminating the fundus of the examinee's eye by irradiating infrared light; focus information obtaining means that is arranged to obtain focus information based on luminance information at a predetermined position on a fundus image obtained by photographing the fundus, illuminated by the illumination light irradiating optical system, by use of the fundus photographing optical system; and focusing means that is arranged to make focusing of the device by moving a focusing lens of the fundus photographing optical system in an optical axis direction based on the focus information obtained by the focus information obtaining means.
(4) In the fundus photographing device (3), the illumination light irradiating optical system includes a focus chart placed in a position which is in an irradiation optical path of the infrared light and is conjugated with the fundus of the examinee's eye, and the focus information obtaining means obtains the focus information based on luminance information of a focus index projected on the fundus image by the focus chart.
(5) In the fundus photographing device (4), the focus chart is movable in the back and forth directions along an optical axis in synchronization with the focusing lens.
(6) In the fundus photographing device (5), the illumination light irradiation optical system includes an optical system for irradiating visible light to the fundus of the examinee's eye, the focus chart is configured to allow the visible light to pass through the entire focus chart, and configured to mask the infrared light at a region for forming the focus index but allow the infrared light to pass through other regions.

### Advantageous Effects of Invention

According to the invention, the device enables easy position adjustment with respect to an examinee's eye and can photograph an eye fundus appropriately.

### Brief Description of Drawings

FIG. 1 is a schematic configuration view of a fundus photographing device in a preferred embodiment;
FIG. 2 is a view of optical systems of the fundus photographing device in the embodiment;
FIG. 3 is a view showing a configuration of a focus chart;
FIG. 4 is a view showing an anterior segment image on which an alignment index is formed;
FIG. 5A is a view for explaining focusing of the fundus photographing device in the embodiment;
FIG. 5B is a view graph for explaining the focusing of the fundus photographing device in the embodiment;
FIG. 6 is a flowchart for analyzing presence/absence of Diabetic Retinopathy by use of a neural network;
FIG. 7 is a schematic view showing a fundus image of a patient's eye with Diabetic Retinopathy;
FIG. 8 is a graph showing luminance distribution information of an entire fundus image from which blood vessels and optic papilla have been removed; and
FIG. 9 is a view showing the fundus image partitioned into local luminance distribution informations.

### Reference Signs List

1 Ophthalmic photographing device
2 Controller
10 Light source
14 Focus chart
26 Two-dimensional light-receiving element
31 Two-dimensional light-receiving element
32a, 32b Light source
100 Photographing part
101 Driving part

### Description of Embodiments

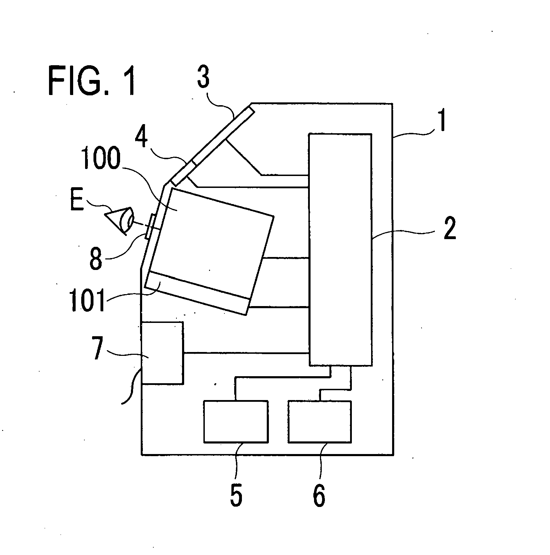

A detailed description of a preferred embodiment of the present invention will now be given referring to the accompanying drawings. FIG. 1 is a schematic configuration view of a fundus photographing device of the present embodiment. The fundus photographing device 1 is provided with a controller 2 including a control circuit having a CPU and others, a monitor 3 for displaying various information, a command input part 4 for inputting various settings, a memory 5 for storing a fundus image and others, an image analyzer 6 for analyzing the stored fundus image by a neural network technique, an output part 7 for outputting an analysis result, a photographing part 100 having an optical system for photographing a fundus of an examinee's eye E, a driving part 101 for driving the photographing part 100 in back and forth, up and down, and right and left directions (in XYZ directions) relative to the examinee's eye E, and others. Numeral 8 denotes a photographing window, before which the examinee's eye E is located for photographing of the fundus of the examinee's eye E by the photographing part 100 installed in the device 1. The monitor 3, command input part 4, memory 5, image analyzer 6, output part 7, photographing part 100 (a light source, a light-receiving element, etc.), and driving part 101 are electrically connected to the controller 2 and will be drivingly controlled in response to a command signal from the controller 2.

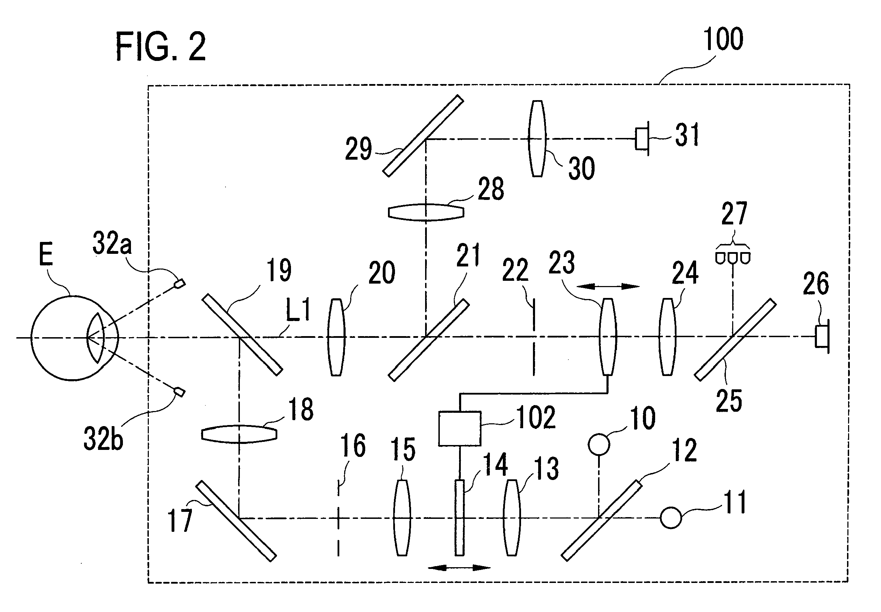

FIG. 2 is a view showing a configuration of optical systems of the photographing part 100.

An optical system for illuminating the examinee's eye E comprises a light source 10 that emits infrared light for fundus illumination, a light source 11 that emits flash light of a visible range for photographing the fundus, a dichroic mirror 12 having the property of reflecting infrared light and allowing visible light to pass, a collimator lens 13, a focus chart 14, a condenser lens 15, a ring slit 16 having a ring-shaped aperture, a mirror 17, a relay lens 18, and a half mirror 19. The light sources 10 and 11 are in conjugate relation with a pupil of the examinee's eye E. The light source 11 can be any light source capable of emitting visible light and, in the present embodiment, it is a light source that emits monochromatic green light to emphasize a blood vessel image in a fundus image to be photographed.

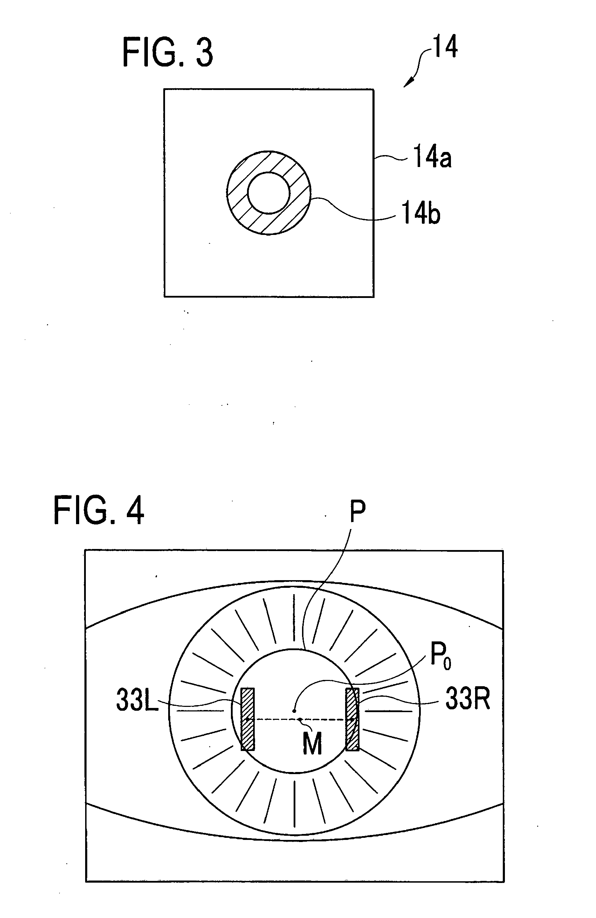

As shown in FIG. 3, the focus chart 14 is formed with a ring-shaped chart 14b of a predetermined size on a filter 14a allowing visible light and infrared light to pass. This chart 14b is formed by a coating capable of allowing passage of visible light and disallowing passage of infrared light. Preferably, this is formed in a sufficiently larger shape than the thickness of blood vessels to distinguish an index from the blood vessels and so on. The focus chart 14 can be moved along an optical axis together with a focusing lens 23 mentioned later by a driving means 102 and form a ring image on the fundus of the examinee's eye E. The ring image serves as an index in focusing. The ring slit 16 is placed in a position conjugated with the pupil of the examinee's eye E through the relay lens 18.

The infrared light emitted from the light source 10 is reflected by the dichroic mirror 12 and passes through the collimator lens 13 to illuminate the focus chart 14 from behind. The infrared light passing through the focus chart 14 passes through the condenser lens 15 to illuminate the ring slit 16. The infrared light passing through the ring slit 16 is reflected by the mirror 17 and then reflected by the half mirror 19 via the relay lens 18, and forms an image on the pupil of the examinee's eye E, thereby illuminating the fundus while forming a ring image on the fundus, which will serve as a focus index. The visible light (the monochromatic green light in the present embodiment) emitted from the light source 11 passes through the dichroic mirror 12 and then travels along the same optical path as the aforementioned infrared light from the light source 10 and illuminates the fundus of the examinee's eye E. Since the chart 14b formed on the focus chart 14 allows visible light to pass, the visible light emitted from the light source 11 illuminates the fundus uniformly without forming a ring image on the fundus of the examinee's eye E.

An optical system for photographing the fundus of the examinee's eye E comprises, in the order closer to the examinee's eye E, the half mirror 19, an objective lens 20, a dichroic mirror 21, an aperture diaphragm 22, the focusing lens 23, an imaging lens (24), a half mirror 25, and a two-dimensional light-receiving element 26. The two-dimensional light-receiving element 26 is in conjugate relation with the fundus of the examinee's eye E. The aperture diaphragm 22 is placed in a position conjugated with the pupil of the examinee's eye E through the objective lens 20. The focusing lens 23 can be moved along an optical axis together with the focus chart 14 by the driving means 102. Reflection light of the illumination light from the fundus by the light source 10 or the light source 11 forms an image once via the half mirror 19 and the objective lens 20 and then is received by the two-dimensional light-receiving element 26 via the dichroic mirror 21, the aperture diaphragm 22, the focusing lens 23, the imaging lens 24, and the half mirror 25.

Numerals 32a and 32b are light sources for projecting, from front of the examinee's eye E, alignment indexes for detecting up and down, right and left, and back and forth directions and for illuminating an anterior segment of the examinee's eye E. The light sources 32a and 32b are a pair of rectangular LEDs disposed symmetrically with respect to a photographing optical axis L1 and emit infrared light of different wavelength from that of the aforementioned light source 10. Each light source 32a and 32b projects an index of diverged light (a rectangular index vertically extending relative to the examinee's eye) at finity, at a predetermined projection angle toward a cornea of the examinee's eye E and illuminates the entire anterior segment.

An optical system for photographing the anterior segment of the examinee's eye E shares the half mirror 19, the objective lens 20, the dichroic mirror 21 with the optical system for photographing the fundus and comprises a field lens 28, a mirror 29, an imaging lens 30, and a two-dimensional light-receiving element 31, which are placed in a direction of reflection of the dichroic mirror 21. An image of the anterior segment of the examinee's eye E illuminated by the light sources 32a and 32b is received, along with the alignment indexes formed on the cornea of the examinee's eye, by the two-dimensional light-receiving element 31 via the half mirror 19, the objective lens 20, the dichroic mirror 21, the field lens 28, the mirror 29, and the imaging lens 30. The two-dimensional light-receiving element 31 is in conjugate relation with the pupil of the examinee's eye E. The dichroic mirror 21 has the property of allowing passage of visible light and the infrared light from the light source 10 while reflecting the infrared light emitted from the light sources 32a and 32b.

Numeral 27 denotes fixation lamps that emit visible light, placed in a direction of reflection of the half mirror 25. This embodiment includes a plurality of fixation lamps, total of nine arranged in 3 x 3, which emit light of the same color (green in this embodiment) as that of the light source 11. When any one of the fixation lamps 27 is selectively turned on for fixation of the examinee's eye E, any one of fundus images different in regions can be obtained.

Operations of the device configured as above will be explained below.

Firstly, an examinee brings his/her face close to the device so that an eye to be photographed (the examinee's eye E) is placed before the photographing window 8 shown in FIG. 1 and the examinee peers through the device. The controller 2 turns on one of the nine fixation lamps 27 (herein, a central one located on the optical axis) to induce fixation. Furthermore, the controller 2 turns on the light sources 32a and 32b and causes the two-dimensional light-receiving element 31 to receive the image of the anterior segment of the examinee's eye E, and makes position adjustment (alignment) between the device (the photographing part 100) and the examinee's eye E based on a light received result thereof.

FIG. 4 is a schematic view showing the anterior segment image received by the two-dimensional light-receiving element 31. When the light sources 32a and 32b are turned on, the anterior segment of the examinee's eye E is illuminated and the rectangular alignment indexes 33L and 33R shown in the figure are projected on the cornea of the examinee's eye E. The controller 2 identifies a pupil P by image processing of the anterior segment image received by the two-dimensional light-receiving element 31 and determines a center Po of the identified pupil P. The controller 2 also obtains centers of the alignment indexes 33L and 33R appearing on the anterior segment image by the image processing and a middle point M of a line joining the centers. An alignment state of the photographing part 100 in the up and down, right and left directions relative to the examinee's eye E is detected from a positional relationship between the determined center Po and the middle point M. An alignment state of the photographing part 100 in the back and forth directions is detected by comparison of an image interval between the alignment indexes 33L and 33R. The alignment indexes 33L and 33R are projected at finity, which will change the image interval as a distance between the examinee's eye E and the photographing part 100 in the back and forth directions changes. In this embodiment, the image interval between the alignment indexes 33L and 33R corresponding to an appropriate alignment distance between the examinee's eye E and the photographing part 100 in the back and forth directions is previously determined as a predetermined value and stored in the memory 5.

As above, the controller 2 obtains distance information for moving the photographing part 100 based on the middle point M determined from the index images (the alignment indexes) formed by the light sources 32a and 32b and the center Po determined as the center of the pupil obtained from the anterior segment image, and drives the driving part 101 to move the entire photographing part 100 in the up and down, right and left directions for alignment so that the middle point M and the center Po coincide with each other. Further, the controller 2 drives the driving part 101 to move the entire photographing part 100 in the back and forth directions relative to the examinee's eye E for alignment so that the interval between the alignment indexes 33L and 33R becomes a predetermined interval (a predetermined value). When the alignment states fall within respective predetermined permissible ranges, the controller 2 determines that the alignment is completed.

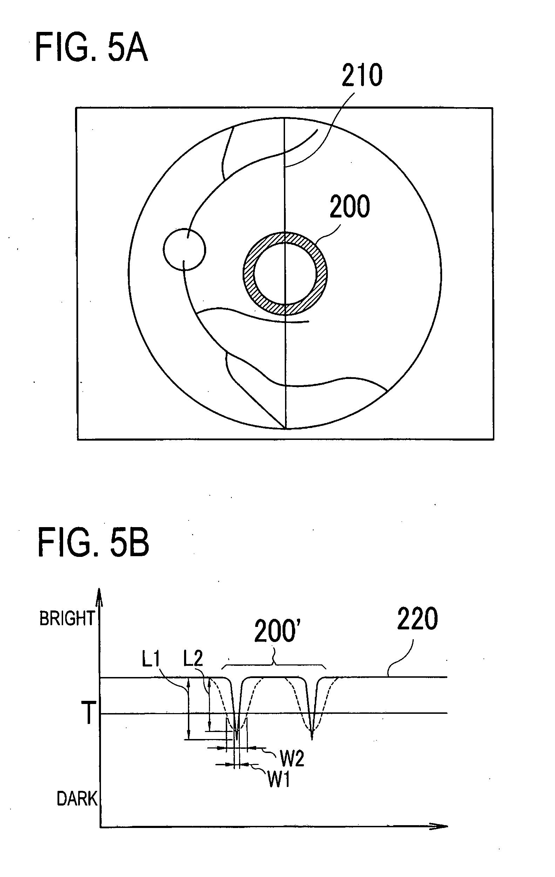

The controller 2 then turns off the light sources 32a and 32b and also turns on the light source 10 for fundus illumination to irradiate the infrared light to the fundus of the examinee's eye E. The reflection light thereof is received by the two-dimensional light-receiving element 26 and thus the fundus image is obtained. FIG. 5A is a schematic view showing the fundus image received by the two-dimensional light-receiving element 26. Numeral 200 denotes an index projected on the fundus by the focus chart 14.

The controller 2 sets a line 210 passing the index 200 on the fundus image received by the two-dimensional light-receiving element 26 and detects luminance information corresponding to the index 200 from luminance information on the set line 210. FIG. 5B is a graph showing luminance information 220 on the set line 210. In the figure, a vertical axis indicates a luminance value and a horizontal axis indicates a position, in which numeral 200' represents the luminance information corresponding to the index 200. In FIG. 5B, for easier explanation, luminance information corresponding to other portions of the fundus such as blood vessels is eliminated. Herein, in the case where a focus state of the photographing part 100 relative to the fundus of the examinee's eye E is not proper, an image of the index 200 projected on the fundus appears blurred. Accordingly, as indicated by a dotted line in FIG. 5B, the luminance information 200' is low in peak height L2 and large in width W2 at a predetermined threshold value T. The controller 2 detects the luminance information 200' corresponding to this index 200 and causes the driving part 102 to move the focus chart 14 and the focusing lens 23 synchronously so that a highest peak height L1 and a smallest width W1 are obtained, and finally determines focus completion. In this embodiment, the index 200 is projected on the fundus by use of the focus chart 14 and the focus is adjusted based on a light-received state (luminance information) of this index 200. However, instead thereof, it may be arranged to extract a specific portion such as a blood vessel from the photographed fundus image and adjust the focus based on luminance information of that portion.

After completion of the alignment and the focus, the controller 2 turns off the light source 10 and turns on the light source 11 to flash the visible light (the monochromatic green light in this embodiment) to illuminate the fundus. The reflection light from the fundus forms an image once via the half mirror 19 and the objective lens 20 and then is received by the two-dimensional light-receiving element 26 via the dichroic mirror 21, the aperture diaphragm 22, the focusing lens 23, the imaging lens 24, and the half mirror 25. The controller 2 stores the obtained fundus image as a fundus image of the examinee's eye E in the memory 5 and turns on other fixation lamps 27 in sequence to obtain more than one fundus image from the same examinee's eye E in the same manner as above.

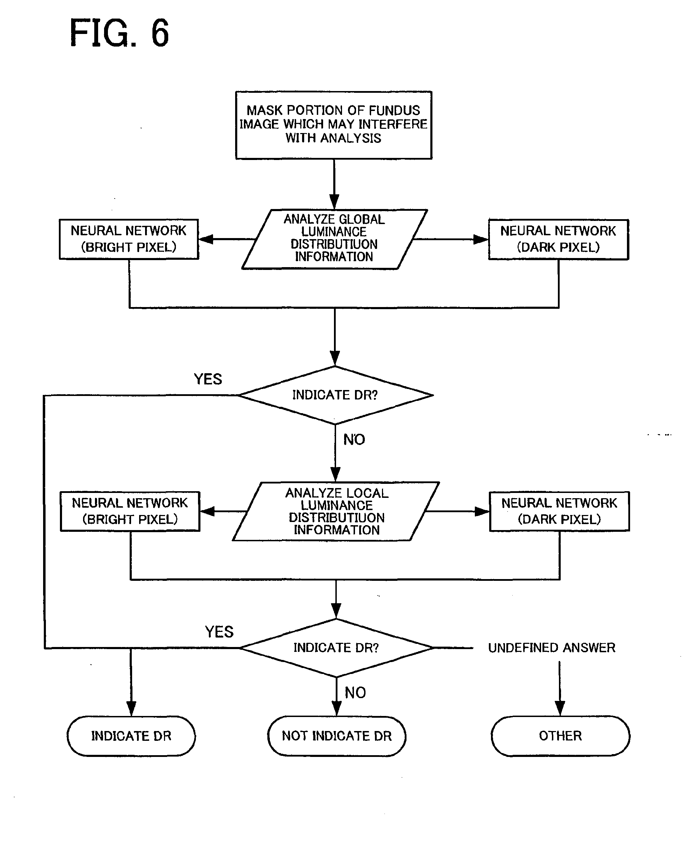

Next, an explanation is given to analysis of the fundus image by the image analyzer 6 with reference to a flowchart in FIG. 6. An ophthalmic photographing device in the present embodiment also serves as a fundus image analysis device capable of analyzing the photographed fundus image by use of a neural network the image analyzer 6 has, and determining whether or not Diabetic Retinopathy (hereinafter, simply abbreviated as DR) has occurred. In the ophthalmic photographing device in the present embodiment, fundus images individually obtained when nine fixation lamps are sequentially turned on are combined into one fundus image by an existing image processing technique and this is analyzed. The following explanation is given to an example of analyzing the fundus image obtained by one fixation lamp for easier explanation.

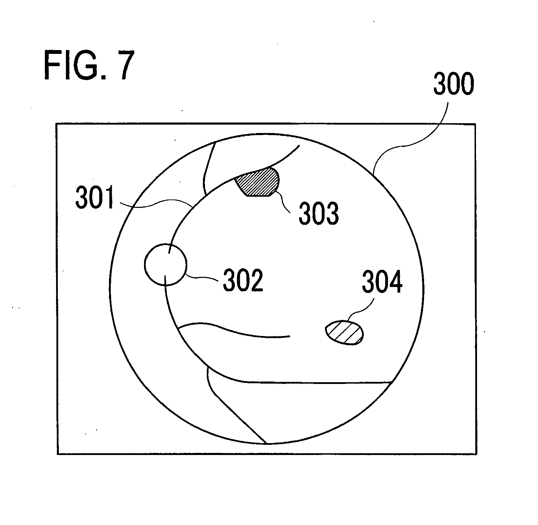

The image analyzer 6 first retrieves a fundus image 300 as shown in FIG. 7 from the memory 5. Such fundus image 300 includes blood vessels 301 and optic papilla 302 (which may not be photographed depending on a position of a turned-on lamp). In the Diabetic Retinopathy (DR) case, a dark color portion 303 resulting from bleeding and a bright color portion 304 called cotton wool spots are photographed. The image analyzer 6 extracts portions such as the blood vessels and the optic papilla, which may interfere with subsequent analysis, in the fundus image by use of the image processing technique, eliminates (masks) corresponding pixels, and counts all of remaining pixels of the fundus image as luminance information of 0 to 255 levels to obtain luminance distribution information of the entire fundus image. It is to be noted that the fundus photographing is performed with the monochrome green light in the present embodiment. Accordingly, red portions such as blood vessels on the fundus are photographed in black color, thus facilitating subsequent extraction of blood vessels and the dark color portion.

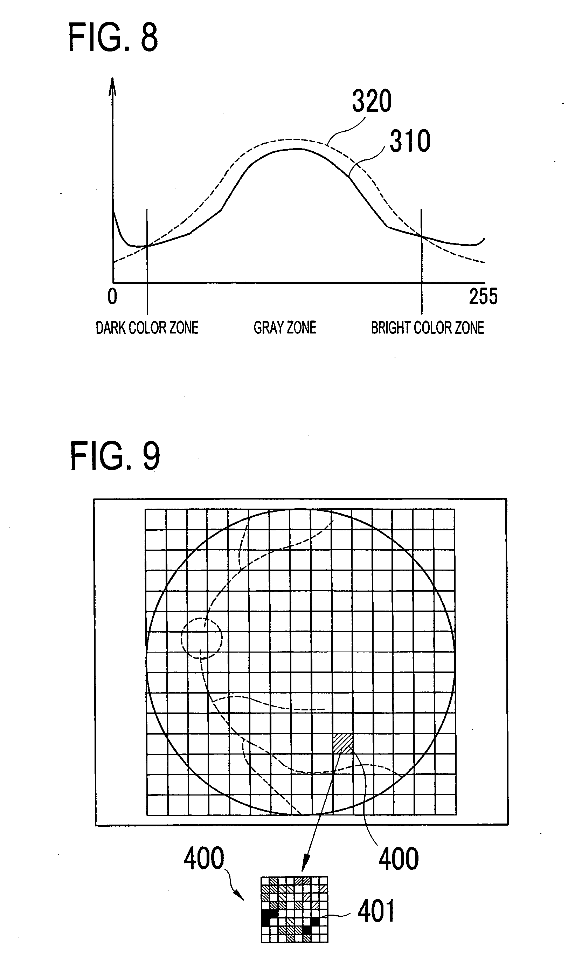

FIG. 8 is a graph showing the luminance distribution information (global luminance distribution information) of the entire fundus image from which the blood vessels 301 and the optic papilla 302 are removed. In the figure, a horizontal axis indicates a luminance value of 0 to 255 and a vertical axis indicates the number of pixels. A solid line 310 shows luminance distribution information based on the fundus image 300 and a dotted line 320 shows previously determined luminance distribution information of a healthy person, which is used as a reference. This luminance distribution information (dotted line) 320 of the healthy person is quantitatively determined from luminance distribution information obtained from fundus images of healthy persons. The image analyzer 6 partitions the luminance distribution information into three zones; a dark color zone, a gray zone, and a bright color zone, by boundaries at which the luminance distribution information (solid line) 310 to be analyzed and the healthy-person's luminance distribution information 320 intersect with each other. The image analyzer 6 analyzes the luminance information of a portion corresponding to the dark color zone in the luminance distribution information 310 by use of the neural network. Similarly, it analyzes the gray zone and the bright color zone and determines whether or not those output results indicate DR.

The neural network in the present embodiment was configured as a feedforward type with three layers; an input layer, a middle layer, and an output layer, and configured to learn (train) input/output data (a teacher signal) for learning by use of a backpropagation method. Characteristics data required for determination of DR such as the total number of pixels in each zone, maximum (minimum) luminance information, a difference in number of pixels between the gray zone and the dark color (bright color) zone, the number of pixels at a peak value in each zone, and the number of pixels at a bottom value in each zone, were given as the input data. In all trainings, if an output error was greater than a desired tolerance, internal weighting parameters of network matrix of neurons were systematically adjusted until an acceptable answer was found. In addition, the network was tested simultaneously with an independent data set after each training through all training map data was previously prepared. As the networks improved to correctly classify both the training and test sets, the acceptable error tolerance between them was set lower in order to achieve higher accuracy.

When the luminance distribution information of the entire fundus image is analyzed by the neural network prepared in the image analyzer 6 and DR is indicated, the controller 2 displays to that effect on the monitor 3. If DR is not indicated (no abnormality is determined), the image analyzer 6 then makes analysis using a neural network based on local luminance information. The neural network used in this case also has the aforementioned configuration and has been subjected to the aforementioned training.

FIG. 9 shows a view showing the fundus image partitioned into local luminance distribution informations. Similarly to the above, the image analyzer 6 masks pixels corresponding to the blood vessels and the optic papilla on the fundus image and partitions the fundus image into small sectors 400. The image analyzer 6 classifies pixels 401 in each sector 400 including no masked pixel into dark, gray, bright pixels and counts the number thereof. If the number of dark or bright pixels exceeds a predetermined value, the sector 400 is counted as abnormal. Every sector 400 with no masked pixel is similarly analyzed to detect any abnormal sector 400.

As to the sector 400 determined to be abnormal, the image analyzer 6 analyzes a portion composed of dark pixels and a portion composed of bright pixels by the same neural network as above and determines whether or not each sector 400 determined to be abnormal indicates DR based on the analysis results. When the image analyzer 6 analyzes the fundus image in each predetermined sector by the neural network and indicates DR, the controller 2 displays (informs) to that effect on the monitor 3. If the output result of the neural network does not indicate DR, the controller 2 displays to that effect on the monitor 3. When it is impossible to determine whether or not DR is indicated by the neural network, an output representing undefinition is made. Based on this output result, the controller 2 determines that another cause is conceivable and displays a result representing Other on the monitor 3. Those display results are printed (informed) through the output part 7 by operation of the command input part 4.

It is to be noted that the present embodiment is achieved by analyzing the fundus image by use of the neural network and determining the presence/absence of occurrence of the DR has occurred from the analysis result, but it is not limited thereto. When distribution tendency of luminance of each pixel is obtained after removal (masking) of the blood vessels and the optic papilla from the fundus image as mentioned above, the use of the neural network enables the same analysis on even different eye diseases based on a local difference in the entire fundus image or in the fundus.

In the present embodiment, the fundus photographing device includes a configuration for image analysis, but not limited thereto. The aforementioned image analysis can also be applied to a device having no function of photographing a fundus and arranged to analyze a fundus image obtained by another device.

## Claims

1. A fundus photographing device including a photographing part (100) having a fundus photographing optical system (19-2d) for photographing a fundus of an examinee's eye (E), comprising:
an alignment index projecting means (32a, 32b) that includes first infrared light sources (32a, 32b) for alignment index projection placed symmetrically with respect to a photographing optical axis (L1) of the fundus photographing optical system and is arranged to project a pair of alignment indexes onto an anterior segment of the examinee's eye;
an anterior segment photographing means (19-21, 28-31) that is arranged to photograph the anterior segment on which the pair of alignment indexes are projected by the alignment index projecting means;
a pupil center detecting means (2, 6) that is arranged to determine a center (P0) of a pupil of the examinee's eye by means of an image of the anterior segment photographed by the anterior segment photographing means;
an alignment index detecting means (2 ,6) that is arranged to determine a middle point (M) of the pair of alignment indexes and an interval between the pair of alignment indexes by means of the anterior segment image photographed by the anterior segment photographing means;
an alignment information obtaining means (2) that is arranged to obtain alignment information of the photographing part in up and down, and left and right directions relative to the examinee's eye from a positional relationship between the detected pupil center and the detected middle point and obtain alignment information of the photographing part in back and forth directions relative to the examinee's eye from the detected interval;
alignment means (2, 101) that is arranged to make alignment of the photographing part with respect to the examiner's eye based on the alignment information obtained by the alignment information obtaining means, the alignment means including a driving part (101) for moving the photographing part in the up and down, left and right, and back and forth directions and a control part (2) for controlling the driving part; **characterized in that**
an illumination light irradiating optical system (10, 12-19) including a second infrared light source (10) for illumination that emits infrared light having a different wavelength from infrared light emitted from the first infrared light sources and a focus chart (14) to pass the infrared light emitted from the second infrared light source to the fundus through the focus chart and project a focus index on the fundus;
a focus information obtaining means (2) arranged to obtain focus information of the photographing part with respect to the fundus based on luminance information of the focus index in an image of the fundus obtained by photographing the fundus, illuminated by the illumination light irradiating optical system, by use of the fundus photographing optical system, the luminance information including peak height (L1, L2) and width (W1, W2) of luminance of the focus index in the fundus image photographed by the fundus photographing optical system; and
a focusing means arranged to make focusing of the photographing part with respect to the fundus based on the focus information obtained by the focus information obtaining means,
the focusing means including a focus lens (23) placed in the fundus photographing optical system, a driving part (102) for moving the focus lens in the back and forth directions along the photographing optical axis, and the control part (2) for controlling the driving part (102) based on the focus information, and the focusing means being arranged to move the focus lens to make the focusing of the photographing part with respect the fundus.

2. The fundus photographing device according to claim 1, wherein the focusing means includes a driving part (102) for moving the focus chart in the back and forth directions along an optical axis of the illumination light irradiating optical system in synchronization with the focusing lens and a control part (2) for controlling the driving part based on the focus information, and
the control part arranged to store the photogaphed fundus image in a memory (5) after completion of the alignment and the focusing and to obtain a plurality of images of the fundus of the same examinee's eye by sequentially turning on other fixation lamps (27).

## Patentansprüche

1. Fundus-Fotogerät mit einem Fototeil (100) mit einem optischen Fundus-Fotosystem (19-26) zum Fotografieren eines Fundus eines Patientenauges (E), aufweisend:
eine Ausrichtungsindex-Projektionseinrichtung (32a, 32b), welche erste Infrarotlichtquellen (32a, 32b) zur Ausrichtungsindexprojektion enthält, die bezüglich einer optischen Fotoachse (L1) des optischen Fundus-Fotosystems symmetrisch angeordnet sind, und ausgestaltet ist, um ein Paar Ausrichtungsindizes auf ein vorderes Segment des Patientenauges zu projizieren;
eine Fotoeinrichtung für das vordere Segment (19-21, 28-31), welche ausgestaltet ist, um das vordere Segment zu fotografieren, auf welches das Paar von Ausrichtungsindizes durch die Ausrichtungsindex-Projektionseinrichtung projiziert wird;
eine Pupillenzentrum-Erfassungseinrichtung (2, 6), welche ausgestaltet ist, um ein Zentrum (P0) einer Pupille des Patientenauges mittels eines Bildes des durch die Fotoeinrichtung für das vordere Segment fotografierten vorderen Segments zu bestimmen;
eine Ausrichtungsindex-Erfassungseinrichtung (2, 6), welche ausgestaltet ist, um einen Mittelpunkt (M) des Paares von Ausrichtungsindizes und einen Abstand zwischen dem Paar von Ausrichtungsindizes mittels des durch die Fotoeinrichtung für das vordere Segment fotografierten Bildes des vorderen Segments zu bestimmen;
eine Ausrichtungsinformationsgewinnungseinrichtung (2), welche ausgestaltet ist, um Ausrichtungsinformationen des Fototeils in Oben- und Unten- sowie Links- und Rechts-Richtungen relativ zum Patientenauge aus einer Positionsbeziehung zwischen dem erfassten Pupillenzentrum und dem erfassten Mittelpunkt zu gewinnen und Ausrichtungsinformationen des Fototeils in Hinten- und Vorne-Richtungen relativ zum Patientenauge aus dem erfassten Abstand zu gewinnen;
eine Ausrichtungseinrichtung (2, 101), welche ausgestaltet ist, um eine Ausrichtung des Fototeils bezüglich des Patientenauges basierend auf den durch die Ausrichtungsinformationsgewinnungseinrichtung gewonnenen Ausrichtungsinformationen vorzunehmen, wobei die Ausrichtungseinrichtung ein Antriebsteil (101) zum Bewegen des Fototeils in den Oben- und Unten-, den Links- und Rechts- sowie den Hinten- und Vorne-Richtungen und ein Steuerteil (2) zum Steuern des Antriebsteils enthält,
**dadurch gekennzeichnet, dass**
ein optisches Beleuchtungslicht-Bestrahlungssystem (10, 12-19) eine zweite Infrarotlichtquelle (10) zur Beleuchtung, welche Infrarotlicht mit einer anderen Wellenlänge als das von den ersten Infrarotlichtquellen emittierte Infrarotlicht emittiert, und eine Fokuskarte (14) zum Leiten des von der zweiten Infrarotlichtquelle emittierten Infrarotlichts durch die Fokuskarte zum Fundus und Projizieren eines Fokusindex auf dem Fundus enthält;
eine Fokusinformationsgewinnungseinrichtung (2)ausgestaltet ist, um Fokusinformationen des Fototeils bezüglich des Fundus basierend auf Luminanzinformationen des Fokusindex in einem Bild des Fundus, das durch Fotografieren des durch das optische Beleuchtungslicht-Bestrahlungssystem beleuchteten Fundus gewonnen wird, unter Verwendung des optischen Fundus-Fotosystems zu gewinnen, wobei die Luminanzinformationen Höhe (L1, L2) und Breite (W1, W2) von Maximalwerten der Luminanz des Fokusindex in dem durch das optische Fundus-Fotosystem fotografierten Fundusbildes enthalten; und
eine Fokussiereinrichtung ausgestaltet ist, um ein Fokussieren des Fototeils bezüglich des Fundus basierend auf den durch die Fokusinformationsgewinnungseinrichtung gewonnenen Fokusinformationen vorzunehmen,
wobei die Fokussiereinrichtung eine in dem optischen Fundus-Fotosystem angeordnete Fokuslinse (23), ein Antriebsteil (102) zum Bewegen der Fokuslinse in den Hinten- und Vorne-Richtungen entlang der optischen Fotoachse sowie das Steuerteil (2) zum Steuern des Antriebsteils (102) basierend auf den Fokusinformationen enthält, und wobei die Fokussiereinrichtung ausgestaltet ist, um die Fokuslinse so zu bewegen, dass sie das Fokussieren des Fototeils bezüglich des Fundus vornimmt.

2. Fundus-Fotogerät nach Anspruch 1, bei welchem die Fokussiereinrichtung ein Antriebsteil (102) zum Bewegen der Fokuskarte in den Hinten- und Vorne-Richtungen entlang einer optischen Achse des optischen Beleuchtungslicht-Bestrahlungssystems synchron zur Fokussierlinse sowie ein Steuerteil (2) zum Steuern des Antriebsteils basierend auf den Fokusinformationen enthält, und
das Steuerteil ausgestaltet ist, um das fotografierte Fundusbild nach Abschluss der Ausrichtung und des Fokussierens in einem Speicher (5) zu speichern und mehrere Bilder des Fundus des gleichen Patientenauges durch sequentielles Einschalten anderer Fixationslampen (27) zu gewinnen.

## Revendications

1. Dispositif de photographie de fond d'oeil comportant une partie de photographie (100) comprenant un système optique de photographie de fond d'oeil (19 à 26) destiné à photographier un fond d'oeil d'un oeil du patient (E), comprenant :
un moyen de projection d'index d'alignement (32a, 32b) qui comporte des premières sources de lumière à infrarouge (32a, 32b) afin d'assurer une projection d'index d'alignement, placées symétriquement par rapport à un axe optique de photographie (L1) du système optique de photographie de fond d'oeil et est agencé de manière à projeter une paire d'index d'alignement sur un segment antérieur de l'oeil du patient ;
un moyen de photographie d'un segment antérieur (19 à 21, 28 à 31) qui est agencé de manière à photographier le segment antérieur sur lequel la paire d'index d'alignement est projetée par le moyen de projection d'index d'alignement ;
un moyen de détection de centre de pupille (2, 6) qui est agencé de manière à déterminer un centre (P0) d'une pupille de l'oeil du patient au moyen d'une image du segment antérieur photographié par le moyen de photographie de segment antérieur ;
un moyen de détection d'index d'alignement (2, 6) qui est agencé de manière à déterminer un point milieu (M) de la paire d'index d'alignement et un intervalle entre la paire d'index d'alignement au moyen de l'image de segment antérieur photographiée par le moyen de photographie de segment antérieur ;
un moyen d'obtention d'informations d'alignement (2) qui est agencé de manière à obtenir des informations d'alignement de la partie de photographie suivant des directions verticale et transversale par rapport à l'oeil du patient à partir d'une relation de position entre le centre de pupille détecté et le point milieu détecté et à obtenir des informations d'alignement de la partie de photographie dans la direction longitudinale par rapport à l'oeil du patient à partir de l'intervalle détecté ;
un moyen d'alignement (2, 101) qui est agencé de manière à assurer l'alignement de la partie de photographie par rapport à l'oeil du patient sur la base des informations d'alignement obtenues par le moyen d'obtention d'informations d'alignement, le moyen d'alignement comportant une partie d'entraînement (101) destinée à déplacer la partie de photographie dans les directions verticale, transversale et longitudinale et une partie de commande (2) destinée à commander la partie d'entraînement ; **caractérisé en ce que**
un système optique d'application de lumière d'éclairement (10, 12 à 19) comportant une seconde source de lumière infrarouge (10) destinée à assurer l'éclairement, qui émet de la lumière infrarouge présentant une longueur d'onde différente de celle de la lumière infrarouge émise à partir des premières sources de lumière infrarouge, et un diagramme de focalisation (14) destiné à laisser passer la lumière infrarouge émise à partir de la seconde source de lumière infrarouge vers le fond d'oeil à travers le diagramme de focalisation et projette un index de focalisation sur le fond d'oeil ;
un moyen d'obtention d'informations de focalisation (2) agencé de manière à obtenir des informations de focalisation de la partie de photographie par rapport au fond d'oeil sur la base des informations de luminance de l'index de focalisation sur une image du fond d'oeil obtenue par la photographie du fond d'oeil éclairé par le système optique d'application de lumière d'éclairement, en utilisant le système optique de photographie du fond d'oeil, les informations de luminance comportant la hauteur (L1, L2) et la largeur (W1, W2) de crête de la luminance de l'index de focalisation sur l'image de fond d'oeil photographié par le système optique de photographie de fond d'oeil ; et
un moyen de focalisation agencé afin d'assurer la focalisation de la partie de photographie par rapport au fond d'oeil sur la base des informations de focalisation obtenues par le moyen d'obtention d'informations de focalisation,
le moyen de focalisation comportant une lentille de focalisation (23) placée sur le système optique de photographie de fond d'oeil, une partie d'entraînement (102) destinée à déplacer la lentille de focalisation dans la direction longitudinale suivant l'axe optique de photographie, et la partie de commande (2) afin de commander la partie d'entraînement (102) sur la base des informations de focalisation, le moyen de focalisation étant agencé de manière à déplacer la lentille de focalisation afin d'assurer la focalisation de la partie de photographie par rapport au fond d'oeil.

2. Dispositif de photographie de fond d'oeil selon la revendication 1, dans lequel le moyen de focalisation comporte une partie d'entraînement (102) destinée à déplacer le diagramme de focalisation dans la direction longitudinale suivant un axe optique du système optique d'application de lumière d'éclairement de manière synchronisée avec la lentille de focalisation et une partie de commande (2) destinée à commander la partie d'entraînement sur la base des informations de focalisation, et
la partie de commande est agencée de manière à mémoriser l'image de fond d'oeil photographiée dans une mémoire (5) après achèvement de l'alignement et de la focalisation et à obtenir une pluralité d'images du fond d'oeil du même oeil du patient en activant de manière séquentielle d'autres lampes de fixation (27).
